# EUROPÄISCHE PATENTANMELDUNG

(11) **EP 3 527 696 A1**
(43) Veröffentlichungstag der Anmeldung: **21.08.2019**
(21) Anmeldenummer: 18156606.8
(22) Anmeldetag: 14.02.2018
(51) Int. Cl.: C25B 1/46, C25B 15/08, C01D 3/06, C01D 3/08, C07C 209/78, C07C 68/02

(54) **VERFAHREN ZUR AUFARBEITUNG UND WIEDERVERWENDUNG VON SALZHALTIGEM PROZESSWASSER**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Es wird ein Verfahren zur Aufarbeitung von salzhaltigem Prozesswasser aus chemischen Produktionsprozessen und Wiederverwendung des Salzes beschrieben, das ein Alkalichlorid als Salz in einer Konzentration von mindestens 4 Gew.-% und organische oder anorganische und organische Verunreinigungen enthält, durch eine Kombination aus Vorreinigung sowie Aufkonzentrierung, Kristallisation und Reinigung des Salzes und gegebenenfalls anschließend Verwendung des Salzes in einer Elektrolyse zur Erzeugung von Basischemikalien.

## Beschreibung

Die Erfindung betrifft ein Verfahren zur Aufarbeitung von salzhaltigem Prozesswasser, insbesondere aus einem chemischen Produktionsprozess, z. B. Prozesswasser aus der Herstellung von Methylen-diphenyl-diisocyanaten (MDI), von Polycarbonat über das Lösungspolymerisationsverfahren (LPC) oder von Diphenylcarbonat (DPC) mit dem Ziel der Nutzung des aus dem Prozesswasser gewonnenen Salzes in der Chlor-Alkali (CA)-Elektrolyse.

**MDI** ist ein wichtiger Stoff der Polyurethanchemie und wird großtechnisch meistens durch Phosgenierung der entsprechenden Polyamine der Diphenylmethanreihe gewonnen. Die Herstellung von Polyaminen der Methylen-Diphenyl-Amin-Reihe, im Folgenden auch kurz **MDA** genannt, ist in zahlreichen Patenten und Publikationen beschrieben. Üblicherweise erfolgt die Herstellung von MDA durch Umsetzung von Anilin und Formaldehyd in Anwesenheit saurer Katalysatoren. Üblicherweise wird Salzsäure als saurer Katalysator eingesetzt, wobei der saure Katalysator gemäß dem Stand der Technik zum Ende des Prozesses und vor den abschließenden Aufarbeitungsschritten, beispielsweise der destillativen Entfernung von überschüssigem Anilin, durch Zusatz einer Base, typischerweise wässriger Natronlauge, neutralisiert und somit verbraucht wird. In der Regel erfolgt die Zugabe des Neutralisationsmittels derart, dass sich das erhaltene Neutralisationsgemisch in eine organische, die Polyamine der MDA-Reihe und überschüssiges Anilin enthaltende Phase, und eine wässrige Phase (MDA-Prozesswasser), welche neben Natriumchlorid noch Reste organischer Bestandteile enthält, auftrennen lässt Ein entsprechendes Vorgehen ist beispielsweise in der EP 2 096 102 A1 beschrieben.

Die Herstellung von Polycarbonat über das Lösungspolymerisationsverfahren (LPC) erfolgt üblicherweise durch ein kontinuierliches Verfahren, zunächst durch Herstellung von Phosgen und anschließende Reaktion von Bisphenolen und Phosgen in Gegenwart von Alkalihydroxid und einem Stickstoffkatalysator, Kettenabbrechern, und gegebenenfalls Verzweigern in einem Gemisch aus wässrig-alkalischer Phase und organischer Lösungsmittelphase in der Phasengrenzfläche.

Die Herstellung von Diarylcarbonaten (DPC) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkalihydroxid und einem basischen Stickstoffkatalysator in der Grenzfläche.

Nach der Reaktion wird in beiden Verfahren (LPC, DPC-Herstellung) die organische, das Polycarbonat enthaltene Phase üblicherweise vom NaCl-haltigen Reaktionswasser abgetrennt, mit einer wässrigen Flüssigkeit (Waschwasser) gewaschen und nach jedem Waschvorgang von der wässrigen Phase soweit wie möglich getrennt. Das entstandene NaCl-haltige und mit Restorganika verunreinigte Reaktionswasser kann separat oder in der Mischung mit Waschwasser beispielsweise mit Wasserdampf gestrippt und als LPC- bzw. DPC-Prozesswasser betrachtet werden. Dies ist beispielhaft in der EP 2 229 343 A1 beschrieben.

Die wässrigen Phasen (der Prozesswässer aus der MDA, LPC oder DPC Produktion) weisen einen Natriumchlorid-Gehalt im Bereich von typischerweise 5 und 20 Gew.- % (Prozesswasser) auf und könnten grundsätzlich in der Chlor-Alkali-Elektrolyse (CA-Elektrolyse) weiterverwendet werden, wenn sie nicht durch produktionsbedingte Stoffe verunreinigt wären.

Es wurde gefunden, dass für den Einsatz dieser Prozesswässer in der CA-Elektrolyse es erforderlich ist, bestimmte Grenzwerte an organischen und anorganischen Verunreinigungen im Prozesswasser einzuhalten, um eine Schädigung von Membranen oder Elektroden durch Ablagerungen oder chemische Prozesse zu verhindern. Um die Prozesswässer sicher in der Chloralkali-Elektrolyse - nachfolgend auch kurz als CA-Elektrolyse bezeichnet - einsetzen zu können, muss der Anteil dieser Verunreinigungen reduziert werden. Es wurde jetzt insbesondere gefunden, dass die Konzentration von organischen Verunreinigungen (TOC, total organic carbon) in der Sole vor dem Elektrolyseur den Wert von 5 mg/L möglichst nicht überschreiten sollte. Anorganische Verunreinigungen (Ca, Mg, Si, Mn, Ni, etc.) führen zur Erhöhung der elektrischen Spannung in der CA-Elektrolyse und sollten ebenfalls möglichst weitgehend entfernt werden.

Das Prozesswasser könnte also gereinigt, mit Anoden-Dünnsole aus der CA-Elektrolyse vermischt und durch die Zugabe von zusätzlichem, festem Alkalichlorid-Salz bis zur notwendigen Elektrolyse-Eintrittskonzentration (z.B. auf ca. 310 g/L NaCl im Falle von NaCl-Abwasser) aufgestockt und in die Elektrolyse geführt werden. Es fallen jedoch in der Regel bei den bekannten Produktionsprozessen großvolumige Prozesswasserströme mit einer vergleichsweise geringen NaCl-Konzentration an, sodass in vielen Fällen nur ein Teil des Gesamtabwasserstroms auf diese Weise recycelt werden kann, da ansonsten der CA-Elektrolyse zu viel Wasser zugeführt würde. Eine einfache Konzentrierung der gereinigten Prozesswässer bis zur typischen Elektrolyse-Eintrittskonzentration (z.B. ca. 310 g/L NaCl) würde zwar dazu führen, dass mehr Prozesswasser recycelt werden könnte, allerdings führt dies gleichzeitig zur Akkumulation der nach der Reinigung verbliebenen organischen und anorganischen Verunreinigungen im Konzentrat, sodass ein zusätzlicher Reinigungsschritt notwendig ist, um die Qualität für die CA-Elektrolyse zu erreichen.

Verfahren zur Aufbereitung salzhaltiger Prozesswässer sind in mehreren Patenten beschrieben worden.

Die Patentschrift CN100506783C offenbart ein zweistufiges Extraktionsverfahren von Polymethylen polyphenyl polyamin aus salzhaltigen Lösungen. Der Nachteil des Verfahrens liegt in der hohen Endkonzentration an Polymethylenpolyphenylpolyamin in der Salzlösung nach der Aufbereitung (hohe TOC und TN Werte), sodass nach dieser Methode aufbereiteten Prozesswässer nicht in der CA-Membranelektrolyse verwendet werden können.

Die Reduktion des TOC-Wertes von Prozesswasser aus der MDI-Produktion durch Oxidation bei einem pH-Wert zwischen 12 und 14 mit anschließender Behandlung durch Aktivkohle beschreiben CN100534931C und EP2479149B1. Durch das Verfahren konnten die TOC-Werte auf den Bereich zwischen 6 und 8 mg/L gesenkt werden. Ein Nachteil des Verfahrens liegt jedoch in der Aktivkohlebehandlung. Durch die mögliche Auswaschung von anorganischen Ionen (Ca, Mg, Si, etc.) aus der Aktivkohle verschlechtert sich die Solequalität wieder, sodass anorganische Ionen wieder aufwändig aus der Sole entfernt werden müssen.

Eine mögliche Aufbereitung von MDA-Prozesswasser und Nutzung in der Elektrolyse beschreibt die EP 2 096 102 A1. Die Aufbereitung erfolgt hierbei durch Einstellen auf einen pH Wert kleiner oder gleich 8, durch Strippen des Prozesswassers mit Wasserdampf, nachfolgender Behandlung mit Aktivkohle und Aufkonzentrierung bzw. Aufstockung der Lösung mit Festsalz (NaCl) auf einen Gehalt von größer als 20 Gew.-% NaCl. Durch das Verfahren kann die nach jetzigen Erkenntnissen für die CA-Elektrolyse erforderliche Reinheit des Prozesswassers bezüglich organischer und anorganischer Verunreinigungen nicht erreicht werden. Außerdem wird ein komplettes Recycling des in der Regel großvolumigen Prozesswasserstroms nicht möglich sein, da eine CA-Membranelektrolyse nur eine begrenzte Menge an zusätzlichem Wasser aufnehmen kann.

Die Aufbereitung der Prozesswässer aus der Polycarbonatherstellung ist ebenfalls mehrfach beschrieben worden. Dabei soll die Reinigung der Prozesswässer von organischen Verunreinigungen und Aufkonzentrierung bis zur Sättigung durch Strippung, Aktivkohle und Osmotische Destillation (siehe WO 2017/001513 A1) oder katalytische Oxidation und Eindampfung erreicht werden (siehe US6340736B1). Beide Aufbereitungsverfahren lösen nicht das oben beschriebene Problem, dass ein großvolumiger Prozesswasserstrom vollständig zu einer CA-Membranelektrolyse recycelt werden kann.

Mehrere Schutzrechte beschreiben Eindampfungs- und/oder Kristallisationsverfahren zur Reduktion von organischen und anorganischen Verbindungen aus salzhaltigen Lösungen. Dabei wurde der Fokus auf die Entfernung von anorganischen Verunreinigungen (US20060144715A1), durch die Zugabe von Salzsäure (US9169131B1) oder durch die Nutzung von Infrarotstrahlung (EP0541114A2) gelegt. Die Offenlegungsschrift EP2565159A1 beschreibt ein Verfahren zur Reinigung industrieller salzhaltiger Lösungen von organischen und anorganischen Verunreinigungen durch Umkristallisation. Aufgrund des doppelten Kristallisationsschrittes ist dieses Verfahren besonders nachteilig und energieaufwändig wegen der zweifachen Verdampfung von Wasser.

Ein weiteres Verfahren zur Aufbereitung organisch belasteter salzhaltiger Abwässer ist in CN203295308U dargelegt. Die Aufbereitung erfolgt in wesentlichen durch die elektrochemische Oxidation organischer Verunreinigungen mittels Diamantelektrode mit anschließender Kristallisation des Salzes. Die Qualität des aufbereiteten Salzes entspricht dennoch nicht den Anforderungen der CA-Elektrolyse, da hier eine Reinheit von 99 Gew.-% als ausreichend für die Aufarbeitung beschrieben wird. Ausgehend vom geschilderten Stand der Technik besteht die Aufgabe darin, ein Verfahren bereitzustellen, bei dem salzhaltige Prozesswässer so gereinigt werden und ihr Wassergehalt so reduziert wird, dass sie in einer Chlor-Alkali-Elektrolyse problemlos recycelt werden können ohne die zuvor beschriebenen weiteren technischen Nachteile insbesondere für einen dauerhaften, störungsfreien Betrieb der Elektrolyse.

Gegenstand der Erfindung ist ein Verfahren zur Aufarbeitung und Wiederverwendung von salzhaltigem Prozesswasser aus einem Produktionsprozess, insbesondere aus einem chemischen Produktionsprozess, das ein Alkalichlorid, bevorzugt Natriumchlorid, als Salz in einer Konzentration von mindestens 4 Gew.-% und organische oder anorganische und organische Verunreinigungen enthält, wobei
a) das Prozesswasser zuerst einer oxidativen und/oder adsorptiven Reinigung zur Entfernung von organischen Verunreinigungen unterzogen wird,
b) gegebenenfalls aus dem gereinigten Prozesswasser durch Entfernen von Wasser ein vorkonzentriertes, gereinigtes Prozesswasser, hergestellt wird, bevorzugt wahlweise durch eines oder mehrere der Verfahren: Hochdruckumkehrosmose, Elektrodialyse, Verdunstung, Membrandestillation oder Verdampfung,
c) gegebenenfalls eine Teilmenge des gereinigten Prozesswassers aus Schritt a) bzw. b) mit einer Salzkonzentration von 4 Gew.- % bis 26 Gew.-%, bevorzugt von 7 Gew.-% bis 26 Gew.-% dem Solekreislauf einer Chloralkali-Elektrolyse zugeführt wird,
d) das Prozesswasser aus Schritt a) oder gegebenenfalls aus Schritt b) oder die aus Schritt c) gegebenenfalls verbleibende Restmenge an Prozesswasser durch Entfernen von Wasser weiter konzentriert wird
e) und das Alkalichlorid auskristallisiert und
f) als festes Alkalichlorid von der Mutterlauge abgetrennt und gereinigt wird, gegebenenfalls mittels einer Wäsche gereinigt wird, sodass das feste Alkalichlorid, analysiert nach Lösung in vollentsalztem reinen Wasser mit einer Konzentration von 300 g/L einen TOC-Gehalt von höchstens 1 mg/L aufweist,
g) das feste, gereinigte Alkalichlorid aus Schritt f) dem Solestrom der Chloralkali-Elektrolyse zugeführt wird,
h) die aus der Alkalichlorid-Elektrolyse nach Schritt g) und gegebenenfalls c) erhaltenen Produkte: Chlor, Alkalilauge, bevorzugt Natronlauge, und gegebenenfalls Wasserstoff dem Produktionsprozess wahlweise wieder zurückgeführt werden.

Das neue Verfahren wird bevorzugt angewendet auf Prozesswasser, in dem die Konzentration an Salz, insbesondere an Alkalichlorid, im Prozesswasser vor Schritt a) mindestens 6 Gew.-%, bevorzugt mindestens 8 Gew.-%, besonders bevorzugt mindestens 12 Gew.-% beträgt.

Bevorzugt ist in dem neuen Verfahren das Alkalichlorid Natriumchlorid und die Alkalilauge Natronlauge.

Das in Schritt f) eingesetzte vollentsalzte Wasser weist insbesondere einen TOC von höchstens 0,01 mg/L auf.

Ein für die Durchführung des neuen Verfahrens besonders geeigneter Produktionsprozess, dem das Prozesswasser entnommen wird, ist ein Verfahren zur Herstellung von Polycarbonaten oder von Polycarbonatvorprodukten, insbesondere von Diphenylcarbonat, oder von Isocyanaten, insbesondere von Methylendiisocyanat (MDI), oder von Methylendiphenylamin (MDA) ist.

Die organischen Verunreinigungen, mit denen das Prozesswasser verunreinigt ist das mit dem neuen Verfahren aufgearbeitet wird, sind insbesondere solche Verbindungen, die ausgewählt sind aus der Reihe: Anilin, MDA und seinen Vorläufern, Formaldehyd, Methanol, Phenol, oder weitere, wie unten beschrieben.

Die anorganischen Verunreinigungen, mit denen das Prozesswasser verunreinigt ist, das mit dem neuen Verfahren aufgearbeitet wird, sind insbesondere solche Verbindungen, die ausgewählt sind aus der Reihe: Salze aus Kationen der Metalle: Ca, Mg, Fe, Al, Si, B, Sc, Ba, Ti, Cr, Mn, Ni und Ru in Verbindung mit Anionen, insbesondere solchen ausgewählt aus der Reihe: Cl⁻, Br⁻, F⁻, SiO₄²⁻,SO₄²⁻

Die optionale oxidative Reinigung in Schritt a) zur Entfernung von organischen Verunreinigungen erfolgt bevorzugt durch Behandlung mit Ozon bei einem Start-pH Wert des Prozesswassers der auf mindestens 1 einzustellen ist und einer Temperatur von mindestens 35°C, bevorzugt mindestens 50°C.

In einer besonders bevorzugten Ausführung des neuen Verfahrens beträgt die Menge an Ozon höchstens 2 g Ozon je Liter Prozesswasser.

Eine weitere bevorzugte Variante des neuen Verfahrens ist dadurch gekennzeichnet, dass die adsorptive Reinigung in Schritt a) zur Entfernung von organischen Verunreinigungen mittels Adsorption an Aktivkohle, Adsorberharzen oder Zeolithen erfolgt.

In einer bevorzugten Variante des neuen Verfahrens erfolgt die Reinigung, d.h. Entfernung von organischen Verunreinigungen aus dem Prozesswasser in Schritt a) wahlweise zusätzlich oder alleine mittels elektrochemischer anodischer Umsetzung an einer Diamantelektrode, bevorzugt an einer Bor-dotierten Diamantelektrode.

Die Entfernung von organischen Verunreinigungen in der Vorreinigung nach Schritt a) wird besonders bevorzugt bis zu einem Restgehalt an Verunreinigungen von höchstens 5 mg/L TOC durchgeführt.

Eine weitere bevorzugte Ausführung des neuen Verfahrens ist dadurch gekennzeichnet, dass die optionale Vorkonzentrierung nach Schritt b) bis zu einer Konzentration von höchstens 26 Gew.-% Alkalichlorid im Prozesswasser erfolgt.

Eine andere bevorzugte Variante des neuen Verfahrens ist dadurch gekennzeichnet, dass das bei der Vorkonzentrierung im optionalen Schritt b) anfallende Wasser zur Verdünnung von Alkalilauge, bevorzugt von Natronlauge, für den chemischen Produktionsprozess, aus dem das Prozesswasser entnommen wurde, insbesondere für einen Prozess für die Herstellung von Polycarbonaten, Polycarbonatvorprodukt oder MDA, weiterverwendet wird.

Auch kann in einer anderen bevorzugten Variante des neuen Verfahrens das bei der Aufkonzentrierung und Kristallisation in den Schritten d) und e) anfallende Wasser zur Verdünnung von Alkalilauge, bevorzugt von Natronlauge, für den chemischen Produktionsprozess, insbesondere einen Prozess für die Herstellung von Polycarbonaten, Polycarbonatvorprodukt oder MDA, weiterverwendet werden.

Das bei der Kristallisation im Schritt f) anfallende feste Alkalichlorid wird in einer besonders bevorzugten Ausführung des neuen Verfahrens vor einer Weiterverwendung mittels vollentsalztem Wasser und/oder mittels gereinigter Alkalichloridlösung (TOC-Gehalt bevorzugt höchstens 5 mg/L), bevorzugt im Gegenstrom, zur Reinigung gewaschen.

In einer weiteren besonders bevorzugten Ausführungsform des neuen Verfahrens wird für die optionale Wäsche des festen Salzes in Schritt f) eine gereinigte Alkalichloridlösung aus einem Teilstrom des in Schritt a) gereinigten Prozesswassers verwendet und/oder es wird Wasser verwendet, das bei der optionalen Vorkonzentrierung nach Schritt b) und/oder der Aufkonzentrierung nach Schritt d) oder e) entfernt wird und anfällt.

In einer anderen bevorzugten Ausführung wird für die Wäsche des Salzes in Schritt f) eine Alkalichloridlösung verwendet, für die gereinigtes Alkalichlorid-Salz Salz in Wasser gelöst wird, das bei der Durchführung von Schritt b) und/oder d) entfernt und erhalten wird. Dies hat den Vorteil, dass besonders reine Alkalichloridlösung (z.B. mit einem Gehalt an TOC < 2 mg/L) zur Wäsche eingesetzt wird.

In einer weiteren bevorzugten Ausführung des neuen Verfahrens wird die in Schritt f) vom Alkalichlorid abgetrennte Mutterlauge in zwei Stoffströme geteilt, wobei der eine größere Stroffstrom in die Aufkonzentration gemäß Schritt d) zurückgeführt wird und der andere, höchstens 5 Gew.-% der abgetrennten Mutterlauge betragende, kleinere Teilstrom entsorgt wird. Dies ist insbesondere bei der kontinuierlichen Betriebsweise erforderlich, da sich sonst die im Kreislauf geführte Mutterlauge immer weiter mit Verunreinigungen anreichert.

Besonders bevorzugt ist noch ein Verfahren, dadurch gekennzeichnet, dass bezogen auf 100 Gewichtsteile abgetrenntes Alkalichlorid 5 bis 20 Gewichtsteile Waschflüssigkeit zur optionalen Wäsche des festen Alkalichlorids in Schritt f) eingesetzt werden.

Bei der MDA-Herstellung anfallendes Prozesswasser, das mit dem neuen Verfahren behandelt werden kann, sollte vor einem Einsatz in der Chloralkali-Membranelektrolyse von noch enthaltenen organischen Verunreinigungen befreit werden. Typische mögliche Verunreinigungen sind insbesondere Anilin, MDA und seine Vorläufer, Formaldehyd, Methanol und Spuren von Phenol, wobei Methanol als Verunreinigung des Formaldehyds, Phenol als Verunreinigung des Anilins in den Prozess gelangen kann. Weitere typische Verunreinigungen sind Formiat, Alkohole, Amine, Carbonsäuren und Alkane. Die Gesamtkonzentration der organischen Verunreinigungen variiert je nach Herstellungsverfahren insbesondere zwischen 50 und 100 mg/L TOC. Die MDA-Prozesswässer weisen üblicherweise herstellungsbedingt einen pH-Wert im Bereich von 12 bis 14 auf, und haben eine typische Konzentration von Natriumchlorid im Bereich von 10 Gew.-% bis 15 Gew.-%. Die Temperatur kann im Bereich von 40 bis 60°C liegen.

Bei den möglichen Hauptverunreinigungen des Prozesswassers aus der Polycarbonatproduktion, das mit dem neuen Verfahren behandelt werden kann, handelt es sich typischerweise um Phenol, Bisphenol A, Phenol- und Benzolderivate mit unterschiedlichen Alkyl-Substitutionen sowie halogenierte Aromaten (bspw. Butylphenol, Isopropylphenol, Trichlorophenol, Dibromophenol) sowie um polare, aliphatische Amine und deren Salze (Trimethylamine, Butylamine, Dimethylbenzylamine) sowie quartären Ammoniumverbindungen und deren Salze. Die Prozesswässer aus Diphenylcarbonat (DPC)- und Polycarbonat-Herstellung nach dem Phasengrenzflächenverfahren (kurz LPC-Produktion genannt) weisen herstellungsbedingt üblicherweise einen pH-Wert im Bereich von 12 bis 14 auf, und haben eine typische Konzentration von Natriumchlorid im Bereich von 5 bis 7 Gew.-% (für LPC-Verfahren) und von 14 bis 17 Gew.-% (für DPC-Verfahren) und eine Temperatur von ca. 30°C.

Phenol und seine Derivate, Bisphenol A und weitere hochmolekulare organische Verbindungen werden in der Chlor-Alkali-Elektrolyse chloriert und bilden AOX (adsorbable organic halogen compounds, adsorbierbare organische Halogenverbindungen). Quartären Ammoniumverbindungen und deren Salze sowie alle Amine führen zur Bildung von NCl₃, sowie Spannungsanstieg der Chlor-Alkali-Elektrolysespannung. Anilin und MDA sind leicht oxidierbar in der Chlor-Alkali-Elektrolyse und führen sofort zur Bildung von Anilin Schwarz, welches Membranen und Elektroden verblockt. Formiat führt zu Verunreinigung des Chlors mit CO₂.

Eine besonders bevorzugte Variante des neuen Verfahrens, ist daher dadurch gekennzeichnet, dass die organischen Verunreinigungen Verbindungen sind, die ausgewählt sind aus der Reihe: Anilin, MDA und seinen Vorläufern, Formaldehyd, Methanol, Phenol, oder Bisphenol A, Phenol- und Benzolderivate mit unterschiedlichen Alkyl-Substitutionen sowie halogenierte Aromaten (bspw. Butylphenol, Isopropylphenol, Trichlorphenol, Dibromphenol) sowie polare, aliphatische Amine und deren Salze (Trimethylamine, Butylamine, Dimethylbenzylamine) sowie quartäre Ammoniumverbindungen und deren Salze.

Das Ziel der Vorreinigung gemäß Schritt a) im neuen Verfahren ist das Recycling von salzhaltigem Prozesswasser um eine komplette Entsorgung des Prozesswassers weitestgehend zu vermeiden, dies betrifft sowohl das Alkalichlorid-Salz mit der Möglichkeit seiner Nutzung in der Elektrolyse zur Herstellung von Chlor als auch das Wasser für dessen Wiederverwendung in der chemischen Produktion. Die Prozesswässer beinhalten organische und anorganische Verunreinigungen wie oben im Einzelnen beschrieben, welche entfernt werden sollten. Akkumulation der Verunreinigungen bei einer Kreislaufführung würde sonst zur Herabsetzung der Produktqualität der Herstellungsprozesse und möglichen Beschädigung der Produktionsanlagen führen. Idealerweise sollen während des Recycling-Verfahrens sowohl das im Prozesswasser enthaltene Salz als auch das Wasser die für die Weiterverwendung notwendige Qualität erhalten. Die Entfernung der Verunreinigungen kann auf unterschiedliche Art und Weise und an verschiedenen Stellen im Prozess erfolgen. Idealerweise sollte dabei die meistens nicht zu vermeidende Menge an zu entsorgendem Prozesswasser möglichst minimiert werden. Sowohl Wasser als auch Salz im Prozesswasser sind Wertstoffe zur Wiederverwendung. Die Entsorgung von Prozesswasser ist daher nicht wirtschaftlich.

MDA und Anilin (Bestandteile des MDA-Prozesswassers) wurden als insbesondere schädliche Substanzen für CA-Elektrolyse identifiziert. Um die Akkumulation dieser Substanzen beispielsweise in der CA-Membranelektrolyse zu verhindern, sollten diese nachweislich entfernt oder zerstört werden. Eine Oxidation mit dem Ziel möglichst die Substanzen zu CO₂ und Wasser zu mineralisieren hat sich als bestgeeignete Methode herausgestellt. Zum einen wird dadurch gewährleistet, dass kein Anilin und MDA in die CA-Elektrolyse gelangt. Zum anderen werden auch weitere im MDA-Prozesswasser enthaltene organische Verunreinigungen zu CO₂ und Wasser mineralisiert, sodass die gesamte TOC-Menge und dadurch auch die zu entsorgende Menge minimiert werden können.

Die Reinigung der bei den angewandten MDA-Verfahren anfallenden Alkalichlorid-haltigen Lösung kann separat (Reaktionswasser) oder - wie in DE10 2008 012 037 A1 dargestellt - zusammen mit anderen Wasserströmen (Waschwasser) erfolgen. Bevorzugt werden die bei der MDA-Herstellung anfallenden Wasserströme zusammengefasst und gemeinsam gereinigt.

Ozonolyse ist ein weit verbreitetes Verfahren zur Entkeimung und Desinfektion von Trinkwasser. Das Verfahren wird auch zunehmend in der Abwasserreinigung zur Oxidation von problematischen Mikroverunreinigungen wie Pharmaka, Pflanzenschutzmittel oder Kosmetika eingesetzt, wobei hier das Ziel ist, die organischen Verunreinigungen nur soweit zu oxidieren, dass diese anschließend biologischen Reinigung zugeführt werden können.

Die Wirkmechanismen von Ozon beim Abbau organischer Substanzen bei verschiedenen pH-Werten sind bekannt. Im sauren und neutralen Bereich lagert sich das Ozonmolekül vorwiegend an Doppelbindungen an, nach der anschließenden Hydrolyse wird das Molekül aufgebrochen. Die Reaktion erfolgt selektiv mit unterschiedlichen Abbaugeschwindigkeiten. Dabei entstehen meist organische Säuren und Ketone. Im alkalischen Milieu wird durch die OH-Radikalbildung eine sehr schnelle Oxidation bewirkt. Die entstehenden OH-Radikale besitzen ein freies Außenelektron, das als starkes Oxidationsmittel dient. Die Reaktion erfolgt in diesem Fall unspezifisch. Für die Mineralisierung von organischen Verunreinigungen zu CO₂ und Wasser wird daher in der Literatur eine Oxidation im alkalischen Medium empfohlen. Der Abbau von Anilin wird beispielweise von 58 % bei pH 3 zu 97 % bei pH 11 erhöht, während CSB (Chemischer Sauerstoffbedarf, Summenparameter als Maß für die Summe aller im Wasser vorhandenen, unter bestimmten Bedingungen oxidierbaren, Stoffe) dabei von 31 % zu 80 % entfernt wird (Journal of Chemistry, Volume 2015, Article ID 905921, 6 pages, http://dx.doi.org/10.1155/2015/905921, Degradation Characteristics of Aniline with Ozonation and Subsequent Treatment Analysis). Phenol konnte beispielweise bei pH 9,4 zu 100 % abgebaut werden, während bei pH 3 nur zu 85 % (S. Esplugas et al. / Water Research 36 (2002) 1034-1042, Comparison of different advanced oxidation processes for phenol degradation).

Ozon oxidiert Schadstoffe (z.B. AOX, Adsorbierbare organisch gebundene Halogene). Bei stark salzhaltigen Wässern kann es passieren, dass z.B. Chlorionen zu Chlor aufoxidiert werden und diese mit organischen Verbindungen reagieren und so wieder AOX bilden können.

Die Wasserlöslichkeit und die Halbwertszeit von Ozon sind bekanntermaßen sehr stark temperaturabhängig. So ist die Löslichkeit von Ozon in Wasser bei 45°C beinah null. Die Reaktionsgeschwindigkeit nimmt dagegen bei einem Temperaturanstieg von 10°C um das Zwei- bis Dreifache zu. Generell wird maximal mögliche Temperatur in einem ozonisierten Wassersystem von ca. 40 °C empfohlen, darüber erfolgt der Ozonabbau zu schnell.

Überraschenderweise wurde insbesondere gefunden, dass die Oxidation bei einem pH-Wert kleiner oder gleich 8 sowie einer höheren Temperatur (50-75°C) zur beinahe vollständigen Mineralisierung aller organischen Verunreinigungen geführt hat (Beispiel bei verschiedenen pH und verschiedenen T). Gemäß einem bevorzugten Verfahren wird die Erniedrigung des pH-Wertes mit Salzsäure oder Chlorwasserstoff durchgeführt.

Die Vorreinigung von DPC und LPC Prozesswasser kann insbesondere durch Behandlung mit Aktivkohle bei einem pH-Wert gleich oder kleiner 8 erfolgen, wie aus dem Stand der Technik grundsätzlich bekannt ist (siehe z. B. EP 2 229 343 A1). Alternativ können andere Adsorbenzien (Zeolithe, makro- und mesoporöse künstliche Harze, Zeolithe etc.) verwendet werden.

Die Kristallisation als zusätzliche Reinigungsstufe ist ein wichtiger Prozess im Gesamtverfahren. Bei der Kristallisation und der Durchführung des neuen Verfahrens ist auf folgende Aspekte bevorzugt zu achten:
1) Die Qualität des gereinigten Salzes sollte bevorzugt die für die Elektrolyse erforderlichen TOC-Wert von kleiner 5 mg/L erreichen;
2) Der Rest-TOC-Gehalt sollte besonders bevorzugt keine für die Elektrolyse schädlichen Substanzen beinhalten (diese könnten außerdem im Elektrolyse-Kreislauf akkumulieren);
3) Das im neuen Verfahren abgetrennte Wasser (Schritte b) oder d)) sollte bevorzugt möglichst keine Restverunreinigungen (TOC bevorzugt unter 2 mg/L) aufweisen (z.B. wegen der Gefahr der Ablagerung von TOC-Komponenten im Verdichter, der bei der Eindampfung mit mechanischer Brüdenkompression eingesetzt wird oder wegen der Gefahr der Verunreinigung des Salzes bei der Wäsche in Schritt f)).

Es wurde insbesondere noch gefunden, dass eine Vorreinigung der Prozesswässer aus unterschiedlicher Polymerproduktion aus mehreren Gründen notwendig ist (hier zum Beispiel MDA und DPC Produktion). Zum einen wurde festgestellt, dass während des Kristallisationsprozesses von aufkonzentriertem MDA-Prozesswasser trotz des erreichten Spezifikationswertes von 3,5 mg/L TOC im Kristallisat eine gelbe Verfärbung des Salzes aufgrund der Oxidation von MDA und Bildung von Anilinschwarz aufgetreten ist (Beispiel 1). Des Weiteren weist die Destillatphase von DPC/LPC/MDA-Prozesswasser einen hohen Anteil an organischen Verunreinigungen auf (siehe Beispiele 1 und 2).

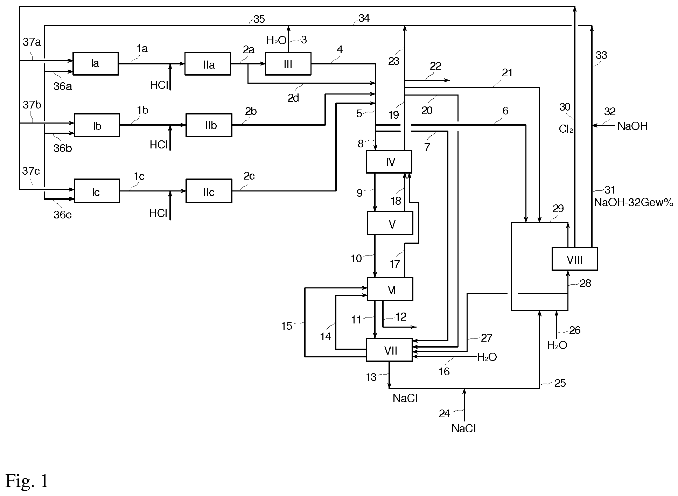

Die Erfindung wird nachstehend anhand der Figur 1 beispielhaft näher erläutert ohne hierauf beschränkt zu sein.

Figur 1 zeigt die schematische Darstellung des erfindungsgemäßen Verfahrens mit Aufkonzentrierung von Prozesswasser aus unterschiedlichen Quellen (MDA-, LPC- und DPC-Produktion) durch Eindampfung und Kristallisation

### Bezugszeichenliste:

- Ia: LPC Produktion
- Ib: DPC Produktion
- Ic: MDA Produktion
- IIa: Adsorptive Vorreinigung
- IIb: Adsorptive Vorreinigung
- IIc: Oxidative Vorreinigung
- III: Vorkonzentrierung
- IV: Wärmetauscher
- V: Eindampfungsstufe
- VI: Kristallisation
- VII: Flüssig/Fest-Separator und Salzwäsche
- VIII: Chloralkali-Membranelektrolyse
- 1a: LPC Prozesswasser
- 1b: DPC Prozesswasser
- 1c: MDA Prozesswasser
- 2a, 2d: Vorgereinigtes LPC Prozesswasser
- 2b: Vorgereinigtes DPC Prozesswasser
- 2c: Vorgereinigtes MDA Prozesswasser
- 3: Entsalztes Prozesswasser aus Vorkonzentrierungsstufe III
- 4: Vorkonzentriertes Prozesswasser aus Vorkonzentrierungsstufe III
- 5: Mischprozesswasser aus 2a, 2b, 2c, 2d und 4
- 6: vorgereinigtes Prozesswasser (Teilstrom)
- 7: vorgereinigtes Prozesswasser (Teilstrom)
- 8: Feedstrom
- 9: vorgewärmte Salzlösung
- 10: eingedampfte Salzlösung
- 11: konzentrierte Salzlösung mit Salz
- 12: Mutterlauge (Purge)
- 13: Salz
- 14: Mutterlauge
- 15: beladenes Waschwasser
- 16: entsalztes Wasser
- 17: Destillat aus Kristallisation VI
- 18: Destillat aus Eindampfung V
- 19: Destillat (Gesamtstrom aus Wärmetauscher IV)
- 20: Destillat (Teilstrom) verwendet als Waschwasser
- 21: Destillat (Teilstrom)
- 22: Destillat (Reststrom)
- 23: Destillat (Teilstrom)
- 24: Festes zusätzliches Salz
- 25: Salzstrom konzentriert
- 26: Wasser
- 27: Salzlösung
- 28: fertige Salzlösung zur Elektrolyse
- 29: Dünnsole
- 30: Chlor
- 31: Natronlauge
- 32: Natronlauge (Zusatz)
- 33: Natronlauge
- 34: verdünnte Natronlauge
- 35: verdünnte Natronlauge
- 36 a, b, c: Natronlauge Eingangsströme zur LPC-, DPC- und MDA-Produktion
- 37 a, b, c: Chlor Eingangsströme zur LPC-, DPC- und MDA-Produktion

### Beispiele:

### Allgemeine Beschreibung der Aufarbeitung und Aufkonzentrierung von Prozesswasser aus verschiedenen Quellen

Die Aufarbeitung und Aufkonzentrierung von Prozesswasser kann durch Eindampfung und Kristallisation der verschiedenen vorgereinigten Prozesswässer separat oder zusammen nach dem Schema wie in Fig. 1 dargestellt erfolgen.

Die Figur 1 gibt eine schematische Darstellung des erfindungsgemäßen Verfahrens mit Aufkonzentrierung von Prozesswasser aus unterschiedlichen Quellen (MDA-, LPC- und DPC-Produktion) durch Eindampfung und Kristallisation wieder.

Bei der LPC-Produktion (Ia) entsteht das Prozesswasser 1a, das zunächst mit Salzsäure (HCl) auf einen pH-Wert kleiner 8 eingestellt, und dann mit Aktivkohle (IIa) vorgereinigt wird. Der vorgereinigte Strom 2a kann optional zu einem Strom 4 vorkonzentriert werden (III) und dem Mischprozesswasser 5 zugeführt werden oder direkt (2d) dem Mischprozesswasser 5 zugeführt werden.

Bei der DPC-Produktion (Ib) entsteht das Prozesswasser 1b, das ebenfalls mit Salzsäure (HCl) auf einen pH-Wert kleiner 8 eingestellt, dann mit Aktivkohle (IIb) vorgereinigt und als Strom 2b dem Mischprozesswasser 5 zugeführt wird.

Bei der MDA-Produktion (Ic) entsteht das Prozesswasser 1c, das auch mit Salzsäure (HCl) auf einen geeigneten pH-Wert eingestellt, dann oxidativ (IIc) vorgereinigt und als Strom 2c dem Mischprozesswasser 5 zugeführt wird.

Dem Mischprozesswasser 5 kann ein Teilstrom 6 entnommen und dem Solekreislauf der Elektrolyse VIII zugeführt werden. Ein weiterer Teilstrom 7 kann optional dem Flüssig/Fest-Separator VII zur Salzwäsche zugeführt werden. Das verbleibende Mischprozesswasser kann dann als Feedstrom 8 dem Wärmetauscher IV zugegeben und in ihm vorgewärmt werden.

Dazu wird bevorzugt das heiße Destillat 18 bzw. 17 aus der Eindampfungsstufe V (Strom 18) oder der Kristallisation VI (Strom 17) verwendet. In der sich anschließenden Eindampfungsstufe V und der Kristallisationsstufe VI wird Wasser als Destillat 17 bzw. 18 durch Verdampfung unter Bildung der Soleströme 9 bzw. 10 entzogen. Die Menge an verdampftem Wasser richtet sich nach der Konzentration von Verunreinigungen im Feedstrom 8. In der Regel können mehr als 95 % des Wassers dem Feedstrom 8 entzogen werden. Je nach Größe des Feedstroms 8 kann es auch sinnvoll sein, Eindampfung und Kristallisation in einem einzigen Apparat durchzuführen (nicht gezeichnet).

Das verdampfte Wasser wird mit Kompressoren verdichtet und zur Beheizung der Eindampfung (Stufe V) bzw. der Kristallisation (Stufe VI) verwendet (Mechanische Brüdenverdichtung; hier nicht gezeichnet). Alternativ kann es aber auch direkt bei mehrstufiger Verfahrensführung in die nächste Stufe einer mehrstufigen Eindampf- bzw. Kristallisationanlage zu Beheizung geleitet werden (hier nicht gezeichnet). Das aus dem Dampf entstehende Kondensat 17 bzw. 18 (Destillat) wird zur Vorwärmung des Feedstroms 8 im Wärmetauscher IV verwendet. Da durch die Vorreinigung des Feedstroms 8 der TOC-Gehalt des Destillats 17 bzw. 18 unter 5 mg/L liegt, kann es nach der Feedvorwärmung in einem, eine besondere Reinheit erfordernden Prozess, z. B. einer ChloralkaliMembran-Elektrolyse VIII, eingesetzt werden (Strom 21).

Bei Eindampfung und Kristallisation VI entsteht ein Gemisch 11 aus festem Salz und mit NaCl gesättigter Mutterlauge. Die Mutterlauge beinhaltet einen Großteil der organischen und anorganischen Verunreinigungen. Daher wird aus der Kristallisationsstufe VI ein Teil der nach der Kristallisation verbleibenden Mutterlauge (Strom 12, Purge), zusammen mit dem Großteil der hierin enthaltenen Verunreinigungen ausgeschleust und verworfen.

Aus dem Gemisch 11 wird im Separator VII ein Teil der Mutterlauge 14 abgetrennt und in den Kristallisationsschritt VI zurückgeführt.

Das feste Salz mit restlicher anhaftender Mutterlauge wird in Stufe VII mit Destillat (Teilstrom 20) als Waschwasser gewaschen und als sauberes Salz 13 erhalten. Dabei ist es besonders vorteilhaft, mit dem Destillat (Strom 20) in der Stufe VII eine Gegenstromwäsche durchzuführen: der besonders reine Strom 20 wird für den zweiten Waschschritt des festen Salzes verwendet. Bei diesem zweiten Waschschritt in Stufe VII nimmt der Strom 20 restliche Verunreinigungen von der Oberfläche des festen Salzes auf. Das beladene Waschwasser wird aufgefangen und für den ersten Waschschritt in Stufe VII verwendet. Dabei verdrängt es die restliche anhaftende Mutterlauge und nimmt zusätzliche weitere Verunreinigungen auf. Da sich das Waschwasser auch mit Salz belädt, wird es als beladenes Waschwasser 15 nach der Wäsche in Stufe VII ebenfalls in die Kristallisation VI zurückgeführt.

Alternativ kann die Wäsche des Salzes in Stufe VII bzw. die Gegenstromwäsche auch mit frischem Wasser, bevorzugt mit entmineralisiertem oder voll entsalztem Wasser (Strom 16) anstelle des Destillats 20 durchgeführt werden.

Da sich das Wasser bei der Wäsche bzw. Gegenstromwäsche in Stufe VII mit Salz belädt, kann wie in der Figur 1 gezeigt optional auch vorgereinigtes Prozesswasser (Strom 7) verwendet werden. Da dieses bereits Salz enthält, ist der Verlust an auskristallisiertem Salz bei der Wäsche geringer.

Die Verwendung der Salzlösung mit Elektrolyse-Eintrittskonzentration (Strom 27) ist besonders vorteilhaft, da sich dabei kaum noch auskristallisiertes Salz löst.

Bezogen auf 100 Gewichtsteile abgetrenntes Alkalichlorid beträgt die Menge der Waschflüssigkeit 10 Gewichtsteile.

Durch das Auskristallisieren und die Gegenstromwäsche wird das gewonnene Salz in einer für die CA-Elektrolyse VIII erforderlichen Reinheit bereitgestellt. Der TOC-Wert beträgt bevorzugt kleiner oder gleich 5 mg/L in der gesättigten Lösung.

Da unter anderem ein Teil des Salzes mit der Mutterlauge (Strom 12) ausgeschleust und verworfen wird, muss zur Bereitstellung einer ausreichenden Chlormenge aus der CA-Elektrolyse (Schritt VIII) für die Prozesse Ia - Ic eine Teilmenge Salz ergänzt werden (Strom 24). Die aus dem Eindampfungs-/Kristallisationsschritt herrührende Salzmenge (Strom 13) und diese Ergänzung 24 werden als Strom 25 der CA-Elektrolyse VIII zugeführt. Je nach den Erfordernissen der Elektrolyse VIII kann die Zufuhr von frischem Wasser (Strom 26) zur Herstellung der Eingangslösung 28 in die Elektrolyse VIII erforderlich sein.

Um den Wasserbedarf der CA-Elektrolyse zu decken kann alternativ ein Teil 6 der vorgereinigten Prozesswässer direkt in die Elektrolyse VIII geführt werden. Der Salzbedarf wird aus dem kristallisierten Salz 13 sowie aus extern zugeführtem Salz 24 gedeckt. Die Ströme werden mit der Dünnsole 29 vermischt sodass eine Solekonzentration von ca. 300 g/L NaCl entsteht. Der TOC-Gehalt in der Mischung darf dabei 5 mg/L nicht überschreiten.

Das in der Elektrolyse entstehende Chlor 30 wird für die Produktionsprozesse von LPC, DPC und MDI verwendet (Chlor Eingangsströme 37a, 37b, 37c). Die entstehende Natronlauge 31 wird dort ebenfalls eingesetzt. Der über die entstehende Natronlauge hinausgehende Bedarf wird gegebenenfalls durch Zufuhr von externer Natronlauge 32 bereitgestellt.

Da der gesamte Natronlaugestrom 33 üblicherweise beim Einsatz in den Produktionsprozessen Ia, Ib und Ic als verdünnte Eingangsströme 36a, 36b, 36c verwendet wird, kann ein Teilstrom 23 des Destillats 19 und das Permeat 3 aus der Vorkonzentrierung zur Herstellung verdünnter Lauge (Ströme 34, 35) eingesetzt werden. Das überschüssige Wasser 22 kann für andere Zwecke in Produktionsprozessen verwendet werden.

### Beispiel 1 (Vergleichsbeispiel)

### Kristallisation ohne Vorreinigung, Salzwasser aus MDA-Produktion:

Es wurde eine belastete Kochsalz-Lösung eingesetzt (Ausganglösung), welche ein typisches MDA-Prozesswasser simuliert, mit folgender Zusammensetzung: 135 g/L NaCl, 132 mg/L Formiat, 0,56 mg/L Anilin, 11,6 mg/L MDA, 30 mg/L Phenol. 1,5 Liter dieser Lösung wurden mit einer Verdampfungsrate von 12 mL/min unter ständigem Rühren ca. 1,155 L Wasser (Destillat) entzogen. Dies entspricht ca. 80 % des Wasseranteils in der Ausganglösung. Das verbliebene den Feststoff enthaltende Konzentrat wurde über eine Nutsche in die Mutterlauge und Festsalz (nass) getrennt. Das abgetrennte feste Salz wurde anschließend mit Reinstsole (reine Waschsole) in der Nutsche gewaschen und aufgefangen (Waschsole). Das gewaschene Salz wurde im Trockenschrank bei ca. 100°C getrocknet. Für die Analysenzwecke wurden anschließend 30 g des getrockneten Salzes mit vollentsalztem reinem Wasser aufgenommen, bis eine Lösung mit der NaCl-Konzentration von 300 g/L (Sole) entstand. In der Tabelle 1 sind die Messwerte der durchgeführten Analysen zusammengefasst. Wie aus der Tabelle 1 ersichtlich ist, wurde durch den Kristallisations- und Waschvorgang der für die Chloralkali--Elektrolyse erforderliche TOC-Wert von 5 mg/L zwar unterschritten, dennoch wurde eine gelbliche Verfärbung von gewonnenem Salz nach dem Trocknungsvorgang beobachtet. Die gelbliche Verfärbung geht zurück auf die Oxidation des MDA. Dies würde für den Einsatz des so gewonnenen Salzes bedeuten, dass die CA-Elektrolyse durch MDA-Oxidationsprodukte auf Dauer geschädigt würde. Des Weiteren ging ein Teil der organischen Verunreinigungen ins Destillat über (TOC 13 mg/L), was eine direkte Wiederverwendung des Destillats in Produktionsprozessen verbieten würde.

**Tabelle 1**

| **MDA ohne Vorreinigung** | Menge | NaCl [g/L] | TOC [mg/L] | pH |
|---|---|---|---|---|
| Ausgangslösung | 1,5 L | 135 | 52 | 12 |
| Destillat | 1,155 L | 0 | 13 | 6,1 |
| Mutterlauge | 265 mL | 310 | 131 | 10,4 |
| Festsalz (nass) | 121,5 g | - | - | - |
| Reine Waschsole | 48 mL | 310 | < 1 | - |
| Waschsole | 48 mL | 310 | 91,5 | - |
| Sole zur Analyse | 100 mL | 300 | 3,5 | 8 |

### Beispiel 2

### Beispiel für alleinige Kristallisation und Wäsche ohne Vorreinigung mit Gegenstromwäsche des erzeugten Salzes, Salzwasser aus DPC-Produktion (Vergleich):

Es wurden 3 Liter DPC-Prozesswasser nach Neutralisation (pH-Wert 7,3) als Vorlage (Ausgangslösung) verwendet. Der Vorlage (DPC-Prozesswasser) wurden mit einer Eindampfrate von 12 mL/min unter ständigem Rühren ca. 2,679 L Wasser (Destillat) entzogen. Dies entspricht ca. 95 % des Wasseranteils im Blank. Das verbliebene Konzentrat wurde durch Nutschen in die Mutterlauge und festes Salz (nass) getrennt. Danach wurde eine zweistufige Gegenstromwäsche mit Waschsole durchgeführt.

Bei der Gegenstromwäsche wird reine Waschsole mit dem bereits einmal gewaschenen Salz in Kontakt gebracht, so dass dieses ein zweites Mal gewaschen wird. Das dann aus der reinen Waschsole entstehende Filtrat wird zur ersten Wäsche des Salzes wiederverwendet.

Dieses prinzipielle Vorgehen wurde folgendermaßen angenähert:

Das abgetrennte feste Salz wurde in zwei etwa gleich große Teilmengen getrennt (Salz S1 und Salz S2). Reine Waschsole wurde ebenfalls in zwei gleich große Teile (reine Waschsole RW1 und reine Waschsole RW2) aufgeteilt. Anschließend wurde das Salz S1 mit reiner Waschsole RW1 auf der Nutsche gewaschen. Das Filtrat wurde als Waschsole WS1.1 aufgefangen. Waschsole WS1.1 stellt damit eine Annäherung an das Filtrat dar, das zur ersten Wäsche des Salzes wiederverwendet wird. Deswegen wurde danach das Salz S2 mit der Waschsole WS1.1 gewaschen, woraus als Filtrat die Waschsole WS1.2 resultierte. Zum Schluss wurde die reine Waschsole RW2 zum erneuten Waschen von Salz S2 verwendet, sodass eine Waschsole WS2 entstand. Das einmal gewaschene Salz S1 und das zweimal gewaschene Salz S2 wurden im Trockenschrank bei ca. 100°C getrocknet. Für die Analysenzwecke wurden anschließend jeweils 30 g vom getrockneten Salz S1 und von getrocknetem Salz S2 in vollentsalztem Wasser auf 100 mL Lösung aufgenommen, so dass eine Sole mit 300 g NaCl/L entstand. In der Tabelle 2 sind die Messwerte der verschiedenen Fraktionen zusammengefasst. Die Qualität der hergestellten Solen So1 und So2 erwies sich als vergleichsweise gut. Es wurde bei dem Versuch dennoch eine hohe Menge an TOC im Destillat (ca. 80% der TOC-Fracht) festgestellt. Dieser Versuch zeigte, dass die **alleinige Kristallisation und Wäsche** des entstandenen Salzes nicht ausreicht, um Destillat in ausreichender Qualität bereitzustellen, die eine Wiederverwendung, wie in Fig. 1 erlaubt.

**Tabelle 2**

| **DPC ohne Vorreinigung** | Menge | NaCl [g/L] | TOC [mg/L] | pH |
|---|---|---|---|---|
| Ausgangslösung | 3L | 177 | 25 | 7,3 |
| Destillat | 2,679 L | 1,5 | 22,5 | 6,8 |
| Mutterlauge | 124 mL | 300 | 99,8 | 9,7 |
| Festsalz (nass) | 535 g | - | - | - |
| Salz S1 (nass) | 272 g | - | - | - |
| Salz S2 (nass) | 263 g | - | - | - |
| Reine Waschsole RW1 | 60 mL | 310 | < 1 | - |
| Reine Waschsole RW2 | 60 | 310 | < 1 | - |
| Waschsole WS1.1 | 64 mL | - | - | - |
| Waschsole WS1.2 | 70 | 310 | 51 | 9,6 |
| Waschsole WS2 | 58 | 310 | 27 | 9,6 |
| Sole So1 | 100 mL | 296 | 1,5 | 9,5 |
| Sole So2 | 100 mL | 294 | < 1 | 9,4 |

Die dargestellten Beispiele zeigen, dass eine Vorreinigung der Prozesswässer notwendig ist. Aufgrund der verschiedenen chemischen Natur der Verunreinigungen müssen unterschiedliche Reinigungsverfahren zur Entfernung von Organika aus den Prozesswässern angewendet werden.

### Beispiel 3 (Verfahren zur Vorreinigung gemäß Schritt a) (erfindungsgemäße Stufe IIc))

### Vorreinigung von MDA-Prozesswasser mit Ozon bei verschiedenem pH:

Gemischtes MDA-Prozesswasser 1c (Reaktions- und Waschwasser) mit einem TOC-Gehalt in der Größenordnung von 70mg/l (Werte siehe Tabelle 3 wurde bei verschiedenem pH-Wert einer O₃-Oxidation IIc unterzogen. Als erstes wurde die Ozonolyse von Original-MDA-Prozesswasser mit dem Start-pH-Wert von pH 13,1 durchgeführt. Zwei weitere Proben wurden mittels HCl auf einen pH-Wert 7 und 3,4 eingestellt und ozoniert. Für die Ozonolyse wurden jeweils 3,5 L Prozesswasser in einem doppelwandigen Glasreaktor unter ständigem Rühren zunächst auf die Temperatur von 75°C gebracht. Für die Ozon-Erzeugung wurde ein Ozon-Generator der Firma Anseros COM-AD-01 verwendet. Die Ozon-Generator-Einstellung wurde bei allen Versuchen konstant gehalten: Sauerstoff-Volumenstrom Eintritt 100 L/h; Generatorleistung 80 % (entspricht ca. 3,5 g Ozon pro Stunde). Ozon-Sauerstoffgemisch wurde dem Glasreaktorzugeführt und mit Prozesswasser vermischt. Zur Kontrolle wurden alle 15 Minuten Proben genommen und TOC sowie pH-Wert gemessen. Die wichtigen Parameter und Ergebnisse sind in der Tabelle 3 zusammengefasst.

**Tabelle 3:**

| **Zeit [min]** | **g O₃ / L** | **Start pH 3** | | **Start pH 7** | | **Start pH 13** | |
|---|---|---|---|---|---|---|---|
| | | **pH** | **TOC [mg/L]** | **pH** | **TOC [mg/L]** | **pH** | **TOC [mg/L]** |
| 0 | 0 | 3,4 | 73,5 | 7 | 68,4 | 13,1 | 69,7 |
| 15 | 0,25 | 3,5 | 62,9 | 7,8 | 35,4 | 13,1 | 37,9 |
| 30 | 0,50 | 3,9 | 43,8 | 8 | 18,8 | 13,1 | 21,3 |
| 45 | 0,75 | 6,9 | 23,7 | 8,3 | 10,1 | 13,1 | 14,4 |
| 60 | 1,00 | 7,5 | 14,6 | 8,4 | 7,3 | 13,1 | 11,5 |
| 75 | 1,25 | 7,9 | 8,2 | 8,5 | 5,7 | 13,1 | 10,6 |
| 90 | 1,50 | 8,1 | 5,7 | 8,5 | 5 | 13,1 | 10,4 |
| 105 | 1,75 | 8,3 | 3,2 | 8,6 | 2,8 | 13,1 | 10,1 |
| 120 | 2,00 | 8,3 | 1,9 | 8,7 | 3,15 | 13,1 | 10,5 |

### Beispiel 4 (Verfahren zur Vorreinigung; erfindungsgemäße Stufe IIc)

### Vorreinigung von MDA-Prozesswasser mit O₃ bei verschiedenen Temperaturen:

Gemischtes MDA-Prozesswasser (Reaktions- und Waschwasser) Ic wurde bei unterschiedlicher Temperatur (25°C, 50°C und 75°C) einer O₃-Oxidation IIc unterzogen. Ozonolyse wurde bei einem Start-pH-Wert von 7,7 durchgeführt. Die wichtigen Parameter und Ergebnisse sind in der Tabelle 4 zusammengefasst. Wie aus der Tabelle 4 ersichtlich wird, verläuft die TOC-Abbaurate bei 75°C um ca. Faktor 2 besser als bei 25°C.

**Tabelle 4**

| **Zeit [min]** | **g O₃ / L** | **25°C** | | **50°C** | | **75°C** | |
|---|---|---|---|---|---|---|---|
| | | **pH** | **TOC [mg/L]** | **pH** | **TOC [mg/L]** | **pH** | **TOC [mg/L]** |
| 0 | 0 | 7,7 | 72 | 7,7 | 72 | 7,7 | 72 |
| 15 | 0,25 | 7,9 | 56 | 8,2 | 45 | 8,4 | 48 |
| 30 | 0,50 | 7,9 | 43 | 8,3 | 42 | 8,4 | 28 |
| 45 | 0,75 | 8,0 | 37 | 8,3 | 28 | 8,5 | 21 |
| 60 | 1,00 | 8,0 | 33 | 8,3 | 24 | 8,5 | 17 |
| 75 | 1,25 | 8,0 | 27 | 8,2 | 22 | 8,5 | 13 |
| 90 | 1,50 | 8,0 | 25 | 8,2 | 16 | 8,5 | 12 |
| 105 | 1,75 | 8,0 | 22 | 8,2 | 15 | 8,6 | 11 |
| 120 | 2,00 | 8,1 | 20 | 8,3 | 11 | 8,6 | 9 |

### Beispiel 5 (erfindungsgemäße Stufe VI)

**Kristallisation nach der Vorreinigung von MDA-Prozesswasser:** Es wurde MDA-Prozesswasser 2c (Ausgangslösung) nach der Vorreinigung durch die Ozonolyse IIc (pH-Wert nach der Ozonolyse 8,1) verwendet. 3 Liter MDA-Prozesswasser 2c wurden analog der Vorgehensweise im **Beispiel 2 (Kristallisation ohne Vorreinigung DPC)** behandelt. In der nachstehenden Tabelle 5 sind die Messwerte der Prozessstoffe zusammengefasst. Es wurde bei dem Versuch eine geringe Menge an TOC im Destillat (ca. 7,9 % TOC-Fracht) festgestellt. Die Qualität der hergestellten Solen So1 und So2 erwies sich als hervorragend.

**Tabelle 5**

| **MDA nach Vorreinigung** | Menge | NaCl [g/L] | TOC [mg/L] | pH |
|---|---|---|---|---|
| Ausgangslösung | 3 L | 153 | 11,3 | 8,1 |
| Destillat | 2,693 L | 1 | 1 | 4,9 |
| Mutterlauge | 147 mL | 310 | 161,8 | 9,6 |
| Festsalz (nass) | 433 g | - | - | - |
| Salz S1 (nass) | 216 g | - | - | - |
| Salz S2 (nass) | 217 g | - | - | - |
| Reine Waschsole RW1 | 60 mL | 310 | < 1 | - |
| Reine Waschsole RW2 | 60 mL | 310 | < 1 | - |
| Waschsole WS1.1 | 51 mL | 310 | - | - |
| Waschsole WS1.2 | 46 mL | 310 | 72,7 | - |
| Waschsole WS2 | 59 mL | 310 | 56,5 | - |
| Sole So1: 30 g Salz 1 + vollentsalztes Wasser ad 100 ml | 100 mL | 296 | < 1 | 8,8 |
| Sole So2: : 30 g Salz 2 + vollentsalztes Wasser ad 100 ml | 100 mL | 296 | < 1 | 8,8 |

Überraschenderweise wurde bei dem Versuch außerdem festgestellt, dass auch anorganische Ionen zum größten Teil in der Mutterlauge verbleiben oder sich durch Salzwäsche entfernen lassen (Tabelle 6). Dabei wurden aus den in der Ausgangslösung gemessenen Ionenkonzentrationen die Massen der Ionen in den 3 Liter der verwendeten Ausgangslösung bestimmt und in die Tabelle eingetragen. Aus den gemessenen Ionenkonzentrationen in Sole So2 wurden die Massen der Ionen berechnet, die sich in Salz S1 und Salz S2 nach entsprechender zweifacher Wäsche befinden würden. Dazu wurde das Volumen der Sole So2 entsprechend der Salzmengen Salz S1 + Salz S2 umgerechnet: 100 ml / 30 g * (216 g + 217 g) = 1.443 ml.

**Tabelle 6**

| **Ion** | **Sr** | **Ni** | **Ru** |
|---|---|---|---|
| **Einheit** | **mg** | **mg** | **mg** |
| 3 L Ausgangslösung (153 g/L NaCl) | 0,172 | < 0,03 | < 0,073 |
| Sole 2 (296 g/L NaCl) , umgerechnet auf alles Salz 1 und Salz 2: 100 ml/30 g * (216 g+217 g) = 1.443 ml | 0,075 | < 0,005 | < 0,016 |

### Beispiel 7 (erfindungsgemäße Stufe VI)

### Kristallisation von Salzwasser aus DPC-Produktion nach Vorreinigung:

Es wurde DPC-Prozesswasser Ib (Ausgangslösung) nach der Vorreinigung IIb durch die Aktivkohle (pH-Wert 7,5) verwendet. 3 Liter von DPC-Prozesswasser 2b wurden analog der Vorgehensweise im **Beispiel 2** (Kristallisation ohne Vorreinigung DPC) behandelt. In der Tabelle 7 sind die Meßwerte der Prozessstoffe zusammengefasst. Es wurde bei dem Versuch kein TOC (Messgrenze kleiner 0,5 mg/L) im Destillat festgestellt. Die Qualität der hergestellten Solen So1 und So2 erwies sich als hervorragend.

**Tabelle 7**

| **DPC nach Vorreinigung** | Menge | NaCl [g/L] | TOC [mg/L] | pH |
|---|---|---|---|---|
| Ausgangslösung | 3 L | 169 | ca. 1 | 7,5 |
| Destillat | 2,662 L | 1,5 | nicht nachweisbar | 5,2 |
| Mutterlauge | 164 mL | 310 | 12,1 | 8,9 |
| Festsalz (nass) | 473 g | - | - | - |
| Reine Waschsole RW1 | 60 mL | 310 | < 1 | - |
| Reine Waschsole RW2 | 60 mL | 310 | < 1 | - |
| Waschsole WS1.1 | 55 mL | 310 | - | - |
| Waschsole WS1.2 | 53 mL | 310 | 27 | - |
| Waschsole WS2 | 59 mL | 310 | 7 | - |
| Sole So1 zur Analyse | 100 mL | 296 | nicht nachweisbar | 8,8 |
| Sole So2 zur Analyse | 100 mL | 296 | nicht nachweisbar | 8,8 |

Auch hier wurde bei dem Versuch festgestellt, dass anorganische Ionen zum größten Teil in der Mutterlauge verbleiben oder durch Salzwäsche sich entfernen lassen. In der Tabelle 8 sind die Messwerte zusammengefasst. Dabei wurden wie schon für Tabelle 6 aus den in der Ausgangslösung gemessenen Ionenkonzentrationen die Massen der Ionen in den 3 Liter der verwendeten Ausgangslösung bestimmt und in die Tabelle eingetragen. Aus den gemessenen Ionenkonzentrationen in Sole So2 wurden die Massen der Ionen berechnet, die sich im Festsalz nach entsprechender zweifacher Wäsche befinden würden. Dazu wurde das Volumen der Sole So2 entsprechend der Festsalzmenge umgerechnet: 100 ml / 30 g * 473 g = 1.577 ml.

**Tabelle 8**

| **Ion** | **Sr** | **Ni** | **Ru** |
|---|---|---|---|
| **Einheit** | **mg** | **mg** | **mg** |
| 3 L Ausgangslösung (169 g/L NaCl) | 0,17 | < 0,027 | < 0,073 |
| Sole So2 (296 g/L NaCl) , umgerechnet auf alles Salz: 100 ml/30 g * 473 g = 1.577 ml | 0,124 | < 0,006 | < 0,017 |

### Beispiel 8 (erfindungsgemäße Stufe VI)

### Kristallisation nach der Vorreinigung von MDA-Prozesswasser, Wäsche mit vollentsalztem Wasser:

Es wird MDA-Prozesswasser 2c (Ausgangslösung) nach Vorreinigung durch die Ozonolyse IIc verwendet und analog der Vorgehensweise im **Beispiel 2** (Kristallisation ohne Vorreinigung von Salzwasser aus der DPC-Produktion) behandelt: Der Vorlage (MDA-Prozesswasser) wird unter ständigem Rühren ca. 94% des Wassers als Destillat entzogen. Das verbliebene Konzentrat wird durch Nutschen in die Mutterlauge und festes Salz (nass) getrennt. Danach wird eine zweistufige Gegenstromwäsche mit vollentsalztem Wasser in der letzten Waschstufe durchgeführt.

Bei der Gegenstromwäsche wird vollentsalztes Wasser mit dem bereits einmal gewaschenen Salz in Kontakt gebracht, so dass dieses ein zweites Mal gewaschen wird. Dabei löst sich ein Teil des Salzes im vollentsalzten Wasser, was zu einem Verlust an festem Salz führt. Das dann aus dem vollentsalzten Wasser entstehende, salzhaltige Filtrat wird zur ersten Wäsche des Salzes wiederverwendet. Dieses prinzipielle Vorgehen wurde folgendermaßen angenähert:

Das abgetrennte feste Salz wurde in drei etwa gleich große Teilmengen getrennt (Salz S1, Salz S2 und Salz S3). Salz S3 wurde mit vollentsalztem Wasser RW1 auf der Nutsche gewaschen. Das Filtrat wurde als beladenes Waschwasser WW2 aufgefangen. Das beladene Waschwasser WW2 stellt damit eine Annäherung an das Filtrat dar, das zur ersten Wäsche des Salzes wiederverwendet wird. Allerdings ist diese Annäherung nicht sehr gut, da Salz S3 ja noch nicht vorgewaschen war.

Deswegen wurde danach Salz S2 mit dem beladenen Waschwasser WW2 auf der Nutsche gewaschen, woraus als Filtrat beladenes Waschwasser WW3 resultierte. Das so vorgewaschene Salz S2 wurde nun mit vollentsalztem Wasser RW4 auf der Nutsche gewaschen, wobei als Filtrat beladenes Waschwasser WW5 entstand. Dieses beladene Waschwasser WW5 ist nun eine deutlich bessere Annäherung an ein Filtrat, das zur ersten Wäsche des Salzes verwendet wird, da es mit vorgewaschenem Salz S2 erzeugt wurde.

Zum Schluss wurde nun Salz S1 mit beladenem Waschwasser WW5 auf der Nutsche gewaschen, wobei als Filtrat beladenes Waschwasser WW6 anfiel. Danach wurde das vorgewaschene Salz S1 mit vollentsalztem Wasser RW7 auf der Nutsche gewaschen, wobei als Filtrat beladenes Waschwasser WW8 erhalten wurde.

Die gewaschenen Salze S1, S2 und S3 wurden im Trockenschrank bei ca. 100°C getrocknet. Für die Analysenzwecke wurden anschließend jeweils 30 g vom getrockneten Salz S1, S2 und S3 in vollentsalztem Wasser auf 100 mL Lösung aufgenommen, so dass die Solen So1, So2 und So3 mit 300 g NaCl/L entstanden. In Tabelle 9 sind die Messwerte der verschiedenen Fraktionen zusammengefasst. Die Qualität der so hergestellten Solen So1, So2 und So3 war vergleichsweise sehr gut. Bei dem Versuch wurde aufgrund der erfindungsgemäßen Vorreinigung nur noch eine kleine Menge an TOC im Destillat (ca. 15% der TOC-Fracht) gefunden.

**Tabelle 9**

| **MDA nach Vorreinigung** | Menge | NaCl [g/L] | TOC [mg/L] | pH |
|---|---|---|---|---|
| Ausgangslösung | 3 L | 145 | 9,3 | 7,3 |
| Destillat | 2,674 L | 1,5 | 1,6 | 6,1 |
| Mutterlauge | 155 mL | 314 | 91 | 9,6 |
| Festsalz (nass) | 406,5 g | - | - | - |
| Salz S1 (nass) | 135,8 g | - | - | - |
| Salz S2 (nass) | 135,2 g | - | - | - |
| Salz S3 (nass) | 135,5 g | - | - | - |
| Vollentsalztes Wasser RW1 | 45 mL | - | - | - |
| Waschwasser WW2 | 50 mL | 258 | - | 9,3 |
| Waschwasser WW3 | 51 mL | 313 | 35,1 | 9,0 |
| Vollentsalztes Wasser RW4 | 45 mL | - | - | - |
| Waschwasser WW5 | 49 mL | 304 | - | 9,1 |
| Waschwasser WW6 | 51 mL | 316 | 25 | 8,9 |
| Vollentsalztes Wasser RW7 | 45 mL | - | - | - |
| Waschwasser WW8 | 51 mL | 304 | 13 | 8,8 |
| Sole So1: 30 g Salz 1 + vollentsalztes Wasser ad 100 ml | 100 mL | 299 | < 1 | 8,0 |
| Sole So2: 30 g Salz 2 + vollentsalztes Wasser ad 100 ml | 100 mL | 298 | < 1 | 8,1 |
| Sole So3: 30 g Salz 3 + vollentsalztes Wasser ad 100 ml | 100 mL | 299 | 2,2 | 8,5 |

### Beispiel 9 (erfindungsgemäße Stufe VI)

### Kristallisation einer Mischung von Prozesswasser aus MDA- und DPC-Produktion:

Es wurde eine Mischung aus 80 % DPC-Prozesswasser 2b nach der Vorreinigung durch die Aktivkohle und 20 % MDA-Prozesswasser 2c nach Ozonolyse verwendet. 3 Liter von Mischung wurden analog der Vorgehensweise im **Beispiel 2 (Kristallisation ohne Vorreinigung DPC)** behandelt. In der Tabelle 9 sind die gefundenen Messwerte der Prozessstoffe zusammengefasst. Bezüglich der Entfernung organischer und anorganischer Verunreinigungen durch Kristallisation und Salzwäsche verhält sich die Mischung analog dem Verhalten der Einzel-Prozesswässer. 90 % der organischen Verunreinigungen werden entfernt. Die meisten Verunreinigungen verblieben in der Mutterlauge, die restlichen Verunreinigungen wurden durch die Salzwäsche entfernt.

**Tabelle 9**

| **Mischung: DPC 80% MDA 20%** | Menge | NaCl [g/L] | TOC [mg/L] | pH |
|---|---|---|---|---|
| Ausgangslösung | 3 L | 177 | 4 | 7,9 |
| Destillat | 2,664 L | 1,5 | 1,5 | 6,9 |
| Mutterlauge | 129 mL | 310 | 37 | 9,4 |
| Festsalz (nass) | 516 g | - | - | - |
| Reine Waschsole RW1 | 60 mL | 310 | < 1 | - |
| Reine Waschsole RW2 | 60 mL | 310 | < 1 | - |
| Waschsole WS1.1 | 62 mL | 310 | - | - |
| Waschsole WS1.2 | 60 mL | 310 | 14 | - |
| Waschsole WS2 | 63 mL | 310 | 6 | - |
| Sole So1 | 100 mL | 296 | < 1 | 9,3 |
| Sole So2 | 100 mL | 296 | < 1 | 9,3 |

## Patentansprüche

1. Verfahren zur Aufarbeitung und Wiederverwendung von salzhaltigem Prozesswasser (1a, 1b, 1c) aus einem Produktionsprozess (Ia, Ib, Ic), insbesondere aus einem chemischen Produktionsprozess, das ein Alkalichlorid, bevorzugt Natriumchlorid, als Salz in einer Konzentration von mindestens 4 Gew.-% und organische oder anorganische und organische Verunreinigungen enthält, wobei
a) das Prozesswasser (1a, 1b, 1c) zuerst einer oxidativen und/oder adsorptiven Reinigung (IIa, IIb, IIc) zur Entfernung von organischen Verunreinigungen unterzogen wird, wobei als bevorzugtes Adsorptionsmittel eines oder mehrere der Reihe: Aktivkohle, Adsorberharze und Zeolithe verwendet werden,
b) gegebenenfalls aus dem gereinigten Prozesswasser (2a, 2b, 2c, 2d) durch Entfernen von Wasser (3) ein vorkonzentriertes, gereinigtes Prozesswasser (4), insbesondere bis zu einer Konzentration von höchstens 26 Gew.-% Alkalichlorid im Prozesswasser, hergestellt wird, bevorzugt wahlweise durch eines oder mehrere der Verfahren: Hochdruckumkehrosmose, Elektrodialyse, Verdunstung, Membrandestillation oder Verdampfung,
c) gegebenenfalls eine Teilmenge (6) des gereinigten Prozesswassers (2a, 2b, 2c, 2d; 4) aus Schritt a) bzw. b) mit einer Salzkonzentration von 4 Gew.- % bis 26 Gew.-%, bevorzugt von 7 Gew.-% bis 26 Gew.-% dem Solekreislauf (29) einer Chloralkali-Elektrolyse (VIII) zugeführt wird,
d) das Prozesswasser (2a, 2b, 2c, 2d; 4) aus Schritt a) oder gegebenenfalls aus Schritt b) oder die aus Schritt c) gegebenenfalls verbleibende Restmenge an Prozesswasser durch Entfernen (V) von Wasser weiter konzentriert wird
e) und das Alkalichlorid auskristallisiert (VI) und
f) als festes Alkalichlorid von der Mutterlauge abgetrennt und gereinigt wird (VII), gegebenenfalls mittels einer Wäsche gereinigt wird, sodass das feste Alkalichlorid (13), analysiert nach Lösung in vollentsalztem reinen Wasser mit einer Konzentration von 300 g/L einen TOC-Gehalt von höchstens 1 mg/L aufweist,
g) das feste, gereinigte Alkalichlorid (13) aus Schritt f) dem Solestrom (28) der Chloralkali-Elektrolyse (VIII) zugeführt wird,
h) die aus der Alkalichlorid-Elektrolyse (VIII) nach Schritt g) und gegebenenfalls c) erhaltenen Produkte: Chlor (30), Alkalilauge (31), bevorzugt Natronlauge, und gegebenenfalls Wasserstoff dem Produktionsprozess (Ia, Ib, Ic) wahlweise wieder zurückgeführt werden.

2. Verfahren gemäß Anspruch 1, **dadurch gekennzeichnet, dass** die optionale oxidative Reinigung (IIa, IIb, IIc) in Schritt a) zur Entfernung von organischen Verunreinigungen durch Behandlung mit Ozon bei einem einzustellenden Start-pH Wert des Prozesswassers (1a, 1b, 1c) von mindestens 1 und einer Temperatur von mindestens 35°C, bevorzugt mindestens 50°C erfolgt und wobei die Menge an Ozon bevorzugt höchstens 2 g Ozon/l Prozesswasser (1a, 1b, 1c) beträgt.

3. Verfahren nach einem der Ansprüche 1 oder 2, **dadurch gekennzeichnet, dass** die Reinigung (IIa, IIb, IIc) von organischen Verunreinigungen aus dem Prozesswasser (1a, 1b, 1c) in Schritt a) wahlweise zusätzlich oder alleine mittels elektrochemischer Umsetzung an einer Diamantelektrode, bevorzugt an einer Bor-dotierten Diamantelektrode erfolgt.

4. Verfahren nach einem der Ansprüche 1 bis 3, **dadurch gekennzeichnet, dass** die Reinigung von organischen Verunreinigungen in Schritt a) bis zu einem Restgehalt an Verunreinigungen von höchstens 5 mg/L TOC erfolgt.

5. Verfahren nach einem der Ansprüche 1 bis 4, **dadurch gekennzeichnet, dass** die Konzentration an Salz, insbesondere an Alkalichlorid im Prozesswasser (1a, 1b, 1c) vor Schritt a) mindestens 6 Gew.-%, bevorzugt mindestens 8 Gew.-%, besonders bevorzugt mindestens 12 Gew.-% beträgt.

6. Verfahren nach einem der Ansprüche 1 bis 5, **dadurch gekennzeichnet, dass** das bei der Vorkonzentrierung im optionalen Schritt b) anfallende Wasser (3) zur Verdünnung von Alkalilauge (33; 34), bevorzugt von Natronlauge für den Produktionsprozess (Ia, Ib, Ic), insbesondere für einen Prozess für die Herstellung von Polycarbonaten (Ia), Polycarbonatvorprodukt (Ib) oder MDA (Ic), weiterverwendet wird.

7. Verfahren nach einem der Ansprüche 1 bis 6, **dadurch gekennzeichnet, dass** das bei der Aufkonzentrierung und Kristallisation in den Schritten d) und e) anfallende Wasser (17, 18, 19, 23) zur Verdünnung von Alkalilauge (33), bevorzugt von Natronlauge für den Produktionsprozess (Ia, Ib, Ic), insbesondere einem Prozess für die Herstellung von Polycarbonaten, Polycarbonatvorprodukt oder MDA, weiterverwendet wird.

8. Verfahren nach einem der Ansprüche 1 bis 7, **dadurch gekennzeichnet, dass** das bei der Kristallisation (VI) im Schritt f) anfallende feste Alkalichlorid (13) vor einer Weiterverwendung mittels vollentsalztem Wasser (16) und/oder mittels gereinigter Alkalichloridlösung (7), bevorzugt im Gegenstrom, zur Reinigung gewaschen wird.

9. Verfahren nach einem der Ansprüche 1 bis 8, **dadurch gekennzeichnet, dass** der Produktionsprozess (Ia, Ib, Ic), dem das Prozesswasser (1a, 1b, 1c) entnommen wird, ein Verfahren zur Herstellung von Polycarbonaten oder von Polycarbonatvorprodukten, insbesondere von Diphenylcarbonat, oder von Isocyanaten, insbesondere von Methylendiisocyanat, oder von Methylendiphenylamin (MDA) ist.

10. Verfahren nach einem der Ansprüche 1 bis 9, **dadurch gekennzeichnet, dass** für die optionale Wäsche des festen Salzes (13) in Schritt f) Wasser verwendet wird, das bei der optionalen Vorkonzentrierung nach Schritt b) und/oder der Aufkonzentrierung nach Schritt d) oder e) entfernt wird und anfällt.

11. Verfahren nach einem der Ansprüche 1 bis 10, **dadurch gekennzeichnet, dass** für die Wäsche (VII) in Schritt f) gereinigte Alkalichloridlösung aus einem Teilstrom (7) des in Schritt a) gereinigten Prozesswassers oder aus einem Teilstrom des in Schritt b) aufkonzentrierten, gereinigten Prozesswassers verwendet wird.

12. Verfahren nach einem der Ansprüche 1 bis 11, **dadurch gekennzeichnet, dass** für die Wäsche (VII) in Schritt f) eine Alkalichloridlösung verwendet wird, für die gereinigtes Alkalichlorid-Salz in Wasser gelöst wird, das bei der Durchführung von Schritt b) und/oder d) entfernt und erhalten wird.

13. Verfahren nach einem der Ansprüche 1 bis 12, **dadurch gekennzeichnet, dass** die in Schritt f) vom Alkalichlorid (13) abgetrennte Mutterlauge in zwei Stoffströme geteilt wird, wobei der eine größere Stoffstrom in die Aufkonzentration gemäß Schritt d) zurückgeführt wird und der andere, höchstens 5 Gew.-% der abgetrennten Mutterlauge betragende, kleinere Teilstrom (12) entsorgt wird.

14. Verfahren nach einem der Ansprüche 1 bis 13, **dadurch gekennzeichnet, dass** bezogen auf 100 Gewichtsteile abgetrenntes Alkalichlorid 5 bis 20 Gewichtsteile Waschflüssigkeit zur optionalen Wäsche des festen Alkalichlorids in Schritt f) eingesetzt werden.

15. Verfahren nach einem der Ansprüche 1 bis 14, **dadurch gekennzeichnet, dass** die organischen Verunreinigungen Verbindungen sind, die ausgewählt sind aus der Reihe: Anilin, MDA und seinen Vorläufern, Formaldehyd, Methanol, Phenol, oder Bisphenol A, Phenol- und Benzolderivate mit unterschiedlichen Alkyl-Substitutionen sowie halogenierte Aromaten (bspw. Butylphenol, Isopropylphenol, Trichlorphenol, Dibromphenol etc.) sowie polare, aliphatische Amine und deren Salze (Trimethylamine, Butylamine, Dimethylbenzylamine) sowie quartären Ammoniumverbindungen und deren Salze.

16. Verfahren nach einem der Ansprüche 1 bis 15, **dadurch gekennzeichnet, dass** die anorganischen Verunreinigungen Verbindungen sind, die ausgewählt sind aus der Reihe: Salze aus Kationen der Metalle: Ca, Mg, Fe, Al, Si, B, Sc Ba, Ti, Cr, Mn, Ni und Ru in Verbindung mit Anionen, insbesondere solchen ausgewählt aus der Reihe: Cl⁻ , F⁻, Br⁻, SiO₄²⁻, SO₄²⁻
